# EUROPEAN PATENT APPLICATION

(11) **EP 4 382 147 A1**
(43) Date of publication of application: **12.06.2024**
(21) Application number: 22306818.0
(22) Date of filing: 07.12.2022
(51) Int. Cl.: A61M 5/142, A61M 5/158, A61M 5/24, A61M 5/315, A61M 5/148, A61M 5/28, A61M 5/32, A61M 5/42

(54) **AUTOMATIC MEDICAL INJECTION DEVICE**

(71) Applicant: Becton Dickinson France, 38800 Le Pont de Claix (FR)
(72) Inventor: LE DIMET, Gwenn, 38850 CHARAVINES (FR); GAGLIANO, Julien, 38240 MEYLAN (FR)
(74) Representative: Regimbeau

(57) **Abstract**

The disclosure relates to an automatic medical injection device comprising:
• a flexible reservoir (20) prefilled with a medical solution, the reservoir comprising a needle (25) for injecting the medical solution into a patient's body, and
• a body (10) removably enclosing the reservoir (20), the body comprising:
o a lower surface (19) adapted to be applied against the patient's skin during an injection of the medical solution, said lower surface (19) comprising a throughhole (14) adapted for the passage of the needle, and
o an upper surface (18) opposite to the lower surface,
o a compression device configured for compressing the reservoir (20) to expel the medical solution through the needle (25), and
o an activation button configured to trigger the compression device upon activation by a user.

## Description

### TECHNICAL FIELD

The disclosure relates to an automatic medical injection device.

### TECHNICAL BACKGROUND

An autoinjector is an automatic injection device designed to facilitate automated delivery of a dose of drug to a patient through a hypodermic needle, the injection usually being administered by the patient themselves. An autoinjector includes a reservoir containing a medical solution and works, for example, by delivering an injection automatically upon actuation by the patient by pressing a button.

The autoinjector therefore needs to be designed in a way that the injection is easy to carry out by the patient himself, and as painless as possible. It is desirable to facilitate the injection even to users having disabilities or weakness in their hands in order to maintain the independence or these patients. In addition to the handling of the autoinjector, its robustness and reliability are also important elements.

Electromechanical autoinjectors can further include a connector to a user control interface allowing to input user instructions, and a controller operated motorized mechanism for dispensing liquid drug dosages from a pre-filled reservoir. Such autoinjectors also need a power source such as a battery for driving the motorized mechanism.

In certain cases, it is desirable to control and vary the speed of the injection, or to interrupt the injection for example in case of pain. The speed control should be accessible by the control interface. Conventional autoinjectors, which are typically spring loaded, do not allow a control of the injection speed.

The vast majority of known autoinjectors are disposable devices, which generates large amounts of waste.

Further, autoinjectors are typically designed for one type and size of reservoir, which makes them less versatile with respect to the dosage and the selection of medical solutions.

### SUMMARY OF THE DISCLOSURE

A goal of the present disclosure is to provide an improved autoinjector designed to overcome the drawbacks mentioned above.

To that end, an object of the disclosure is an automatic medical injection device comprising:
- a flexible reservoir prefilled with a medical solution, the reservoir comprising a needle for injecting the medical solution into a patient's body, and
- a body removably enclosing the reservoir, the body comprising:
   ∘ a lower surface adapted to be applied against the patient's skin during an injection of the medical solution, said lower surface comprising a throughhole adapted for the passage of the needle, and
   ∘ an upper surface opposite to the lower surface,
   ∘ a compression device configured for compressing the reservoir to expel the medical solution through the needle, and
   ∘ an activation button configured to trigger the compression device upon activation by a user.

Preferably, the reservoir has an oblong portion along a longitudinal axis, and a tapered portion extends from a distal portion of the oblong portion, and the needle is arranged in a distal tip of the tapered portion.

Advantageously, the compression device comprises a support plate supporting the reservoir, a roller arranged for compressing the reservoir, at least one pinion rack and at least one pinion connected to the roller so that a rotational movement of the pinion on the pinion rack provokes a translational movement of the roller on the reservoir. The direction of the translational movement of the roller is along the longitudinal axis.

The support plate may be moveable between:
- a storage position in which the injection needle is maintained inside the body, and
- an injection position in which the injection needle is protruding from the body for penetrating the patient's skin,
the body comprising a blocking system configured for selectively maintaining the support plate in the storage position or the injection position.

Preferably, the support plate is tiltable around a tilting axis between the storage position and the injection position.

The automatic medical injection device may further comprise at least one holding flap protruding from the upper surface of the body, preferably two holding flaps, said at least one holding flap being configured for at least partially surrounding a hand of a user during injection.

The automatic medical injection device may further comprise a Peltier element adapted for cooling the patient's skin, said Peltier element surrounding the throughhole in the lower surface.

The automatic medical injection device may further comprise a beeper configured for emitting an acoustic signal at the end of the injection.

Advantageously, the body comprises a microprocessor and a motor, the motor being arranged to drive the compression device so as to expel the medical solution through the needle, said motor being controlled by the microprocessor.

Preferably, the motor is configured to drive the pinion to roll on the pinion rack.

The activation button may be configured for starting and/or stopping the motor and/or controlling a speed of the motor.

The automatic medical injection device may further comprise a rechargeable battery adapted for supplying electrical power to at least one of the microprocessor, the motor, the Peltier element, or the beeper, the body further comprising an electrical connector arranged through a wall of the body and configured to connect an electric power source for recharging the rechargeable battery.

The electric connector may further be configured to connect an external electronic device to the microprocessor.

### BRIEF DESCRIPTION OF THE DRAWINGS

Further features and advantages will appear in the following detailed description, based on the appended drawings, wherein:
Figure 1A is a perspective view of the injection device from above.
Figure 1B is a perspective view of the injection device from below.
Figure 2 is a perspective view of a flexible reservoir.
Figure 3 is a perspective view of a support plate.
Figure 4 is a perspective view of an embodiment of a flexible reservoir.
Figure 5A is a perspective view of an embodiment of a support plate.
Figure 5B illustrates a roller bar on a pinion rack.
Figure 6 illustrates one embodiment of a compression device including a pinion and a pinion rack.
Figure 7A schematically illustrates the storage position.
Figure 7B schematically illustrates the injection position.
Figure 8A illustrates the storage position before an automatic injection.
Figure 8B illustrates the beginning of an automatic injection.
Figure 8C illustrates the progression of the automatic injection.
Figure 8D illustrates the final configuration after an automatic injection.

### DETAILED DESCRIPTION OF EMBODIMENTS

In the following description, the terms "proximal" and "distal" are considered in relation to the injection direction of the medical solution. Similarly, the term "distal direction" means the direction of injection, and the term "proximal direction" means the direction opposite to the direction of injection.

### Presentation of the Injection Device

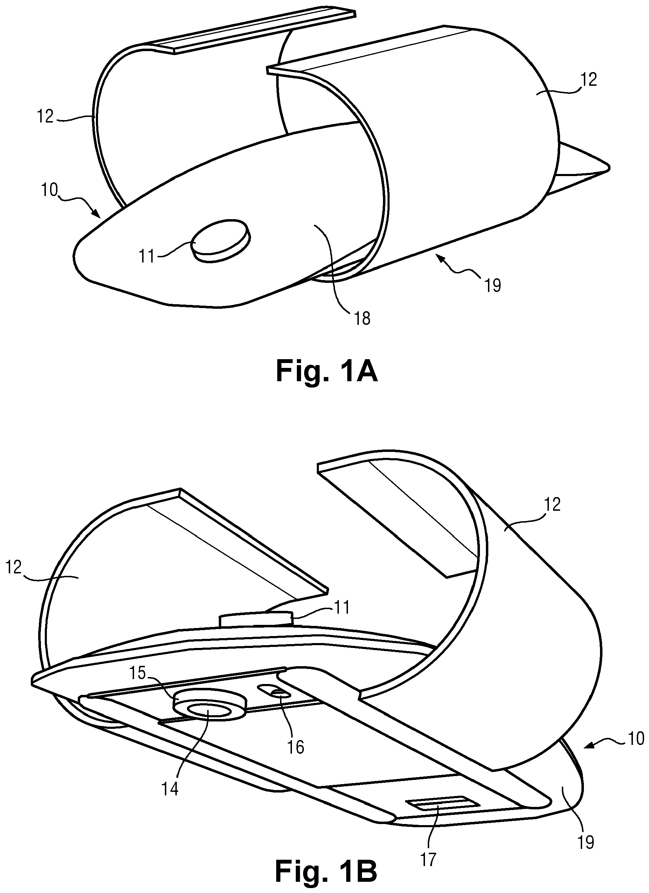

The invention relates to a reusable automatic medical injection device configured for automatically administering an injection to a patient after pressing an activation button. Figure 1A shows the automatic injection device from above. The device comprises a body 10 comprising an upper surface 18 which is held in the palm of the user during the injection. The upper surface 18 is typically slightly convex in order to comfortable fit into the palm of the user and is made of a rigid material providing a comfortable feel in the hand of a user, such as a plastic material. The upper surface 18 further comprises an activation button 11 which is situated in a position which is in easy reach of the fingers of the user during the injection, typically close to the proximal end of the upper surface 18. The activation button 11 is configured to start the injection when the button 11 is pressed. In certain embodiments, the activation button can also be configured for stopping the injection at any moment and/or for slowing down or accelerating the injection speed. Commands for such variations might be given by pressing the button more or less strongly or by sequences in which the button is pressed several times.

As illustrated in figure 1B, the body further comprises a lower surface 19 which is in contact with the skin of the patient during the injection. The lower surface 19 is typically flat to provide a firm support for the injection device and to prevent the device from moving during the injection. However, the invention is not limited to a flat lower surface, the lower surface can for example adopt a shape adapted to the part of the body in which the injection should be performed. The lower surface 19 is made of a rigid material providing a comfortable feel on the skin of the patient, for example a plastic material.

The lower surface 19 comprises a throughhole 14 for the passage of a needle during the injection. The throughhole 14 is located close to the distal end of the injection device.

The automatic injection device further comprises on or more holding flaps 12, preferably two holding flaps 12, which are arranged on the sides of the body 10. The holding flaps 12 are protruding upwards from the lateral sides of the body 10, said lateral sides extending from the distal end to the proximal end. Preferably, the holding flaps 12 are bent for at least partially surrounding a hand of a user during injection.

The body may comprise an upper part and a lower part removably connected to each other so as to allow opening the body. For example, the upper part comprises the upper surface 18 and the lower part comprises the lower surface 19. In some embodiments, the upper part is fully removable from the lower part. In other embodiments, the upper part is pivotably mounted onto the lower part via a hinge.

In the interior, the body 10 is comprising a flexible reservoir which may contain a medical solution, and a compression device for compressing the reservoir. The reservoir is exchangeable and can be removed from the body, for example by opening the body.

The automatic injection device can thus be reused one or several times by removing the empty reservoir and inserting a new prefilled reservoir each time. This allows to reduce cost and waste volume, for example during long-term treatments and for patients having chronical diseases treated by the injections.

### Flexible Reservoir

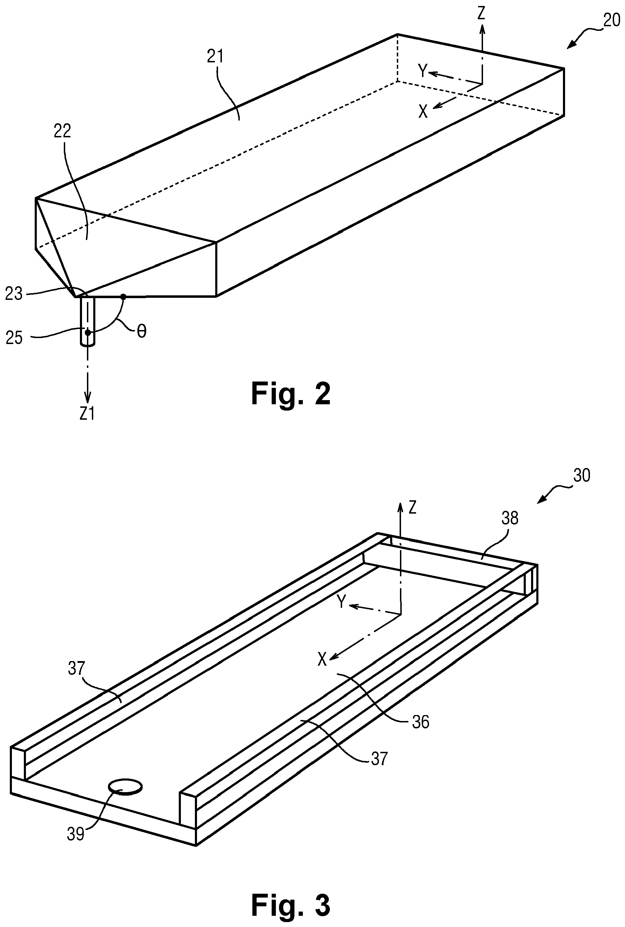

With reference to figure 2, the reservoir 20 has an elongated shape and extends along a longitudinal axis X. The reservoir 20 comprises an oblong portion 21 on its proximal end and a tapered portion 22 on its distal end. The needle 25 is fixed on the distal tip 23 of the tapered portion 22. This arrangement allows to completely empty the reservoir 20 during an injection.

The needle 25 extends along an injection axis Z1 from the tip 23 of the reservoir 20. Preferably, the injection axis Z1 of the needle 25 forms an angle θ with respect to the reservoir 20 in order to point out of the lower surface 19 of the device. The oblong portion 21 of the reservoir 20 is typically aligned with the lower surface of the body of the automatic injection device. The angle θ between the injection axis Z1 of the needle and the lower surface of the body is chosen to allow the needle 25 to easily penetrate the throughhole in the lower surface of the body and prick the skin of the patient..

The reservoir 20 is made of a flexible material, which is preferably inert to the chemical products comprised in the medical solution. Typically, the flexible reservoir 20 is made of a polymer such as HDPE, LDPE, PE, PVDC, PP, or PET. The reservoir 20 can be transparent in order to verify the quantity and condition of the medical solution contained inside. In other embodiments, the reservoir 20 is opaque.

The reservoir can contain different quantities of medical solutions, for example a quantity comprised between 1 ml and 20 ml. In particular, a reservoir of a standard size can be prefilled with either a maximum quantity of medical solution, or any quantity inferior to said maximum quantity. Thus, such a reservoir containing any quantity of medical solution can be used with one single automatic injection device, which makes the device more versatile and allows for example to gradually decrease or increase the amount of medicine administered to a patient in subsequent injections.

### Compression Device

The compression device is configured for deforming the flexible reservoir, hereby flattening the reservoir from the proximal end towards the distal end during the injection. The flattening of the reservoir results in forcing the medical solution towards the distal tip of the reservoir and out of the needle into the body of the patient.

The compression device comprises a support plate configured to support the flexible reservoir. The support plate is preferably arranged between the lower surface of the body and the flexible reservoir. The support plate 30 is made of a rigid material, for example a metal or a rigid plastic material. With reference to figure 3, the support plate 30 presents an essentially rectangular shape and extends along the longitudinal direction X. The support plate can comprise a throughhole 39 for the passage of the needle extending out of the flexible reservoir. In this case, the throughhole 39 of the support plate 30 is aligned with the throughhole of the lower surface of the body in order to form a passage for the needle via the aligned throughholes. The support plate 30 can further comprise two rim strips 37 extending in longitudinal direction along the sides of the top surface 36 of the support plate, whereby said top surface 36 is configured to be in contact with the flexible reservoir. The rim strips 37 border the side of the flexible reservoir and prevent the reservoir from slipping off the support plate 30. An additional rim strip 38 can be provided at the short side on the proximal end of the support plate 30.

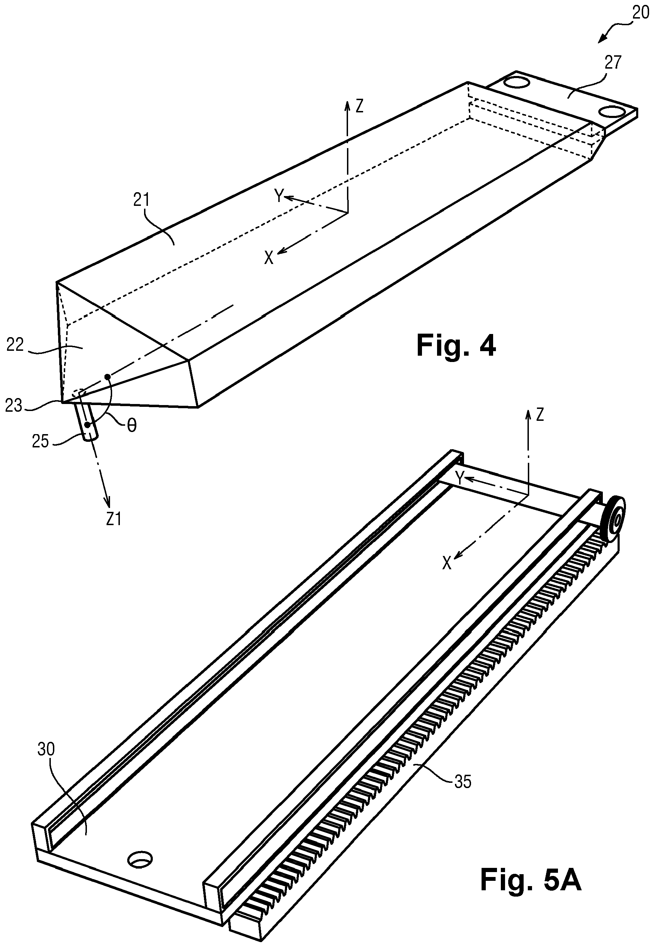

The flexible reservoir can be fixed on the top side 36 of the support plate. In the embodiment shown in figure 4, the flexible reservoir 20 comprises a fixing plate 27 on its proximal end. The fixing plate 27 at the proximal end of the flexible reservoir 20 is attached to the proximal edge of the support plate by a fixing means, for example by clips, pins, a clamp or any other temporary fixing means allowing to remove the flexible reservoir after performing the injection.

The compression device further comprises a roller for compressing the flexible reservoir. The roller has an essentially cylindrical shape defining a transversal axis Y in the plane of the surface of the support plate and perpendicular to the longitudinal axis X.

The compression device preferably comprises a pinion connected to the roller. The pinion can be directly attached to the roller. Alternatively, the pinion can be arranged at a distance to the roller and be connected to the roller by a structure such as an arm, a frame or a bracket. When the pinion performs a rotational movement on the pinion rack, the engagement of the pinion on the teeth of the rack provokes a movement of the roller in a longitudinal direction, resulting in a squeezing movement on the reservoir.

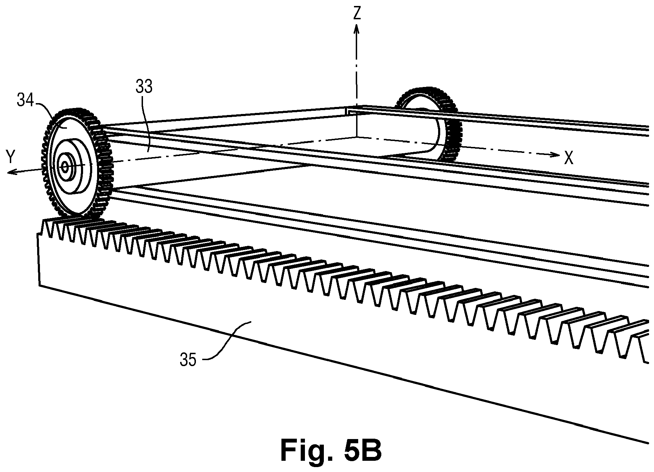

In the embodiment shown in figures 5A and 5B, the compression device comprises a pinion rack 35 arranged in longitudinal direction X on one long side of the support plate 30. The teeth of the pinion rack 35 are facing towards the upper surface of the automatic injection device in a horizontal direction Z. With reference to figure 5B, the compression device further comprises a pinion 34 firmly attached on one end face of the cylindrical roller 33. The teeth of the pinion 34 are engaged with the teeth of the pinion rack 35 so that a rotational movement of the pinion 34 on the pinion rack 35 provokes a translational movement of the roller 33 on the reservoir. The translational movement is carried along the longitudinal axis X in distal direction, and can be reversed in proximal direction in order to change the reservoir and prepare the device for a new injection. The pinion 34 and the pinion rack 35 can be arranged on any side of the support plate 30. Alternatively, the compression device can comprise two pinion racks arranged on either side of the support plate, and two pinions arranged respectively on the two ends of the cylindrical roller.

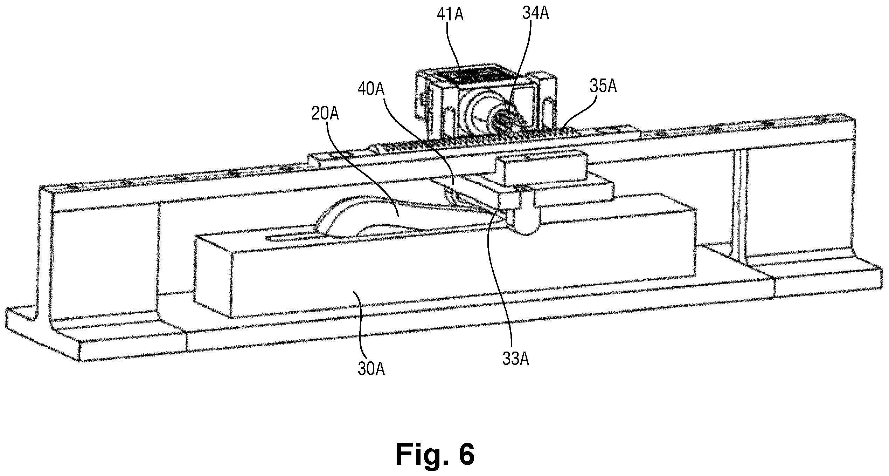

In an alternative embodiment shown in figure 6, the pinion rack 35A, the roller 33A and the pinion 34A are arranged on the side of the flexible reservoir 20A opposite of the support plate 30A. The teeth of the pinion rack 35A are facing away from the flexible reservoir 20A. The flexible reservoir 20A is arranged on the support plate 30A and compressed by the roller 33A. The roller 33A is arranged on an arm 40A extending away from the support plate. The pinion rack 35A is arranged on the side of the roller opposite of the reservoir 20A and the support plate 30A. A pinion 34A is rotatably arranged on the arm 40A and engaged in the pinion rack 35A. In this configuration, the arm 40A can also support a drive motor 41A configured for driving the rotational movement of the pinion 34A.

### Moveable support plate

Advantageously, the injection device can be switched between a storage position in which the injection needle is maintained inside the body, and an injection position in which the injection needle is protruding from the body for penetrating the patient's skin. In this way, needlestick injuries can be prevented before and after use of the injection device.

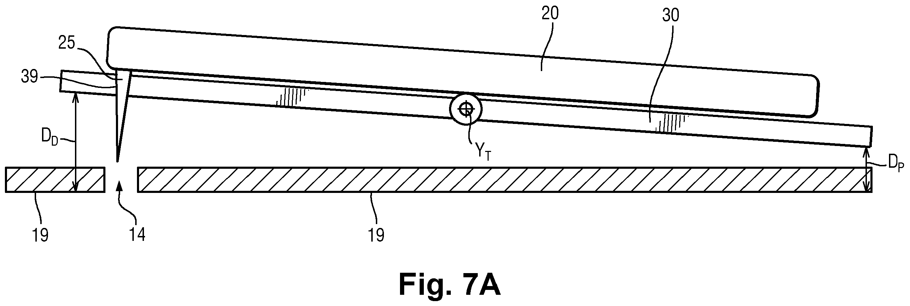

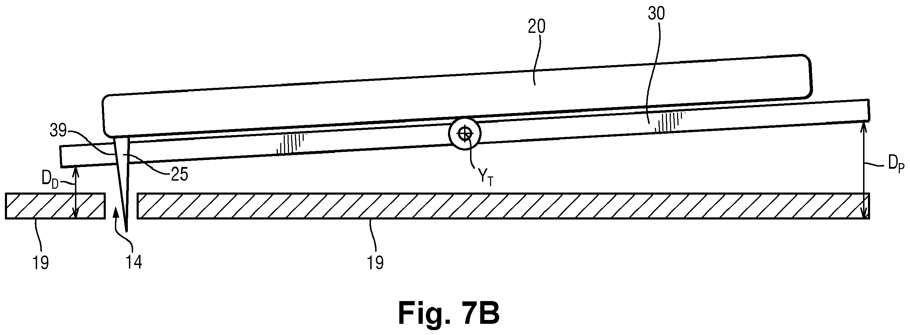

With reference to figures 7A and 7B, the transition between the storage position and the injection position is triggered by pressing the activation button, provoking a tilting movement of the support plate around a tilting axis Y_{T} in the plane of the support plate and perpendicular to the longitudinal axis X.

In the storage position, as illustrated in figure 7A, the distance D_{D} between the distal end of the support plate 30 and the lower surface 19 of the body is greater than the distance D_{P} between the proximal end of the support plate 30 and the lower surface 19 of the body. In this position, the distal end of the reservoir 20 and the needle 25 are set back from the throughhole 14 in the lower surface 19. Hence, the needle 25 is completely contained inside the body of the injection device.

In the injection position, as illustrated in figure 7B, the distance D_{D} between the distal end of the support plate 30 and the lower surface 19 of the body is smaller than the distance D_{P} between the proximal end of the support plate 30 and the lower surface 19 of the body. In this position, the needle 25 extends through the throughhole 14 in the lower surface 19 and is sticks out of the body of the injection device so that the tip of the needle 25 can pierce the patient's skin for injecting the medical solution for subcutaneous injection.

The tilting movement of the support plate is initiated either by pressing the actuation button or by pressing manually the device on the skin of the patient. The movement can be performed by an actuator or motor lifting or pressing down one or both ends of the support plate, or by an actuator provoking a rotational movement of a rod aligned with the tilting axis Y_{T}.

At the end of the injection, the needle retracts automatically by the tilting of the support plate in its storage position.

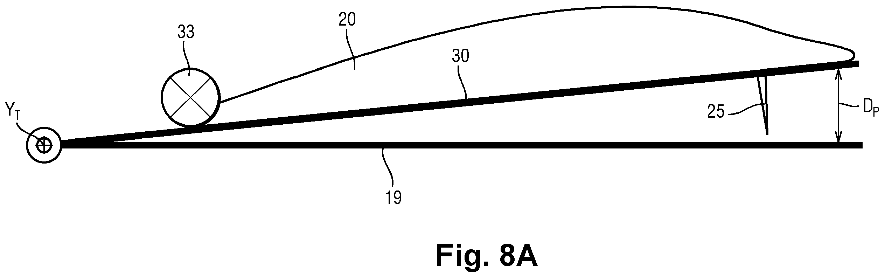

The tilting axis Y_{T} can for example be positioned under the flexible reservoir as shown in figures 7A and 7B, or on the distal end of the support plate as indicated in figures 8A to 8D, or any other position in the body of the automatic injection device.

### Motors

The automatic injection device comprises at least a first motor for provoking the rotational movement of the pinion, for example a stepper motor connected to the pinion via a rotating axis, a fan belt or a pinion belt. The motor is arranged inside the body of the device. The injection device can further comprise a second motor for performing the tilting movement of the support plate around the tilting axis Y_{T}.

### Optional elements

The automatic injection device can further include a Peltier element for cooling the patient's skin during the injection and hereby avoid or attenuate the sensation of pain and swelling. Advantageously, the Peltier element is arranged on the lower surface of the body and surrounding the throughhole through which the injection needle penetrates to the body of the patient. The cooling is typically started at the beginning of the injection and can start before or after the injection device moves into the injection position.

The injection device can further comprise a beeper for signaling the end of injection. The beeper is triggered when the pinion and the roller reach their final position and the total amount of the medical solution is injected. It is possible to program a delay for starting the beeper.

### Microprocessor

The automatic injection device comprises a microprocessor configured to control the one or several motors. When the injection device comprises a Peltier element and/or a beeper, the microprocessor is also used to control these elements. Optionally, the microprocessor can be configured by an external user interface such as a computer or smartphone, for example for setting different injection options like speed, quantity to be injected and for example cooling parameters for the Peltier element and acoustic signals of the beeper at the end of the injection.

The automatic injection device further comprises a battery, preferably a rechargeable battery, for providing electrical power to the one or more motors and the microprocessor and optional elements such as a Peltier element and/or a beeper. The battery can be recharged by connecting an external charging device to an electrical connector 17 which is preferably arranged on the lower surface of the injection device.

When the automatic injection device comprises an electronic connector for configuring the microprocessor, the same electronic connector can be configured for charging the battery, for example a USB connector

The microprocessor can be also used to record patient data such as treatment observance, date and time of injection, injection issues... etc. Data transfer can be done by several means such as USB, Bluetooth or Wi-Fi.

### Use of the automatic injection device

In order to prepare an automatic injection, the support plate is arranged in its storage position. The body is opened and the roller is set to a starting position on the distal end of the support plate. With reference to figure 8A, a prefilled reservoir 20 containing a medical solution is inserted into the body and arranged on the support plate 30. When the reservoir and/or the container comprise fixing means, the prefilled reservoir is fixed to the container using the fixing means. The body is then closed and the device is ready for performing an injection.

The user places his hand on the upper surface of the body, the holding flaps embracing the hand of the user, and positions the injection device on the part of the patient's body in which the injection is to be performed.

The user then pushes the activation button in order to start the injection. When the injection device comprises a Peltier element, the Peltier element is provided with electrical power for cooling the skin of the patient. The support plate is moved from the storage position to the injection position by tilting around the tilting axis. Thereby, the tip of the injection needle extends through the throughhole in the lower surface and pricks the skin of the patient.

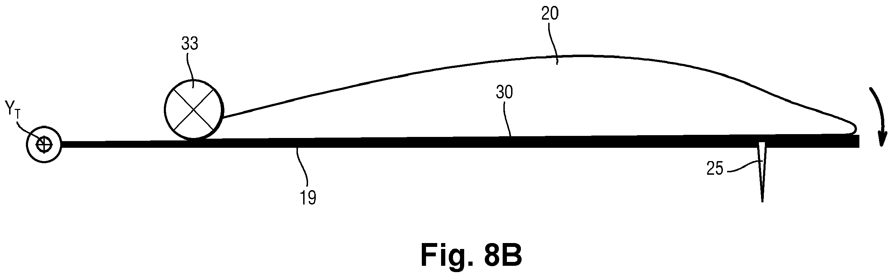

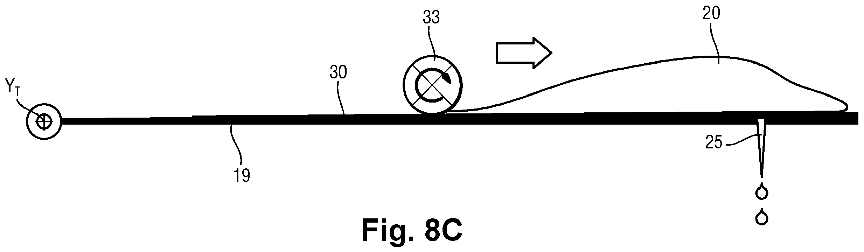

At the beginning of an injection, with reference to figure 8B, the proximal end of the flexible reservoir 20 is compressed between the roller 33 and the proximal end of the support plate 30. During the injection, with reference to figure 8C, the roller rolls 33 over the reservoir 20 in the distal direction, performing simultaneously a rotational movement around the transversal axis Y and a transversal movement along the longitudinal axis X in distal direction. The transversal movement is indicated by an arrow in figure 8C. Hereby, the roller deforms the reservoir by successively compressing the reservoir from the proximal to the distal end. This deformation results in squeezing the medical solution out of the distal tip of the reservoir 20 on which the needle 25 is arranged. The medical solution flows through the needle into the body of the patient.

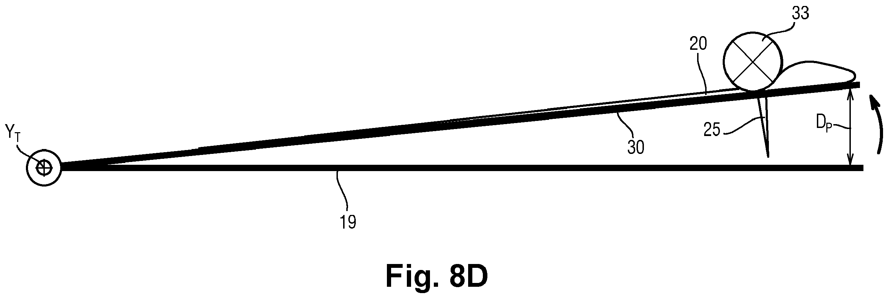

When the roller 33 reaches the distal end of the reservoir 20 as illustrated in figure 8D, the reservoir 20 is flattened on the support plate 30 and the entire amount of medical solution has been expelled from the flexible reservoir 20. The support plate 30 is then moved back into the storage position, retracting the needle tip into the body. The Peltier element is disconnected from the power source and stops cooling. When the injection device comprises a beeper, said beeper emits an acoustic signal that the injection is accomplished.

The user can now remove the injection device from the patient and remove or replace the empty reservoir.

## Claims

1. An automatic medical injection device comprising:
• a flexible reservoir (20) prefilled with a medical solution, the reservoir comprising a needle (25) for injecting the medical solution into a patient's body, and
• a body (10) removably enclosing the reservoir (20), the body comprising:
• a lower surface (19) adapted to be applied against the patient's skin during an injection of the medical solution, said lower surface (19) comprising a throughhole (14) adapted for the passage of the needle, and
• an upper surface (18) opposite to the lower surface,
• a compression device configured for compressing the reservoir (20) to expel the medical solution through the needle (25), and
• an activation button configured to trigger the compression device upon activation by a user.

2. The automatic medical injection device according to claim 1, wherein the reservoir has an oblong portion (21) along a longitudinal axis (X), and a tapered portion (22) extends from a distal portion of the oblong portion (21), and the needle (25) is arranged in a distal tip (23) of the tapered portion (21).

3. The automatic medical injection device according to any or claims 1 to 2, wherein the compression device comprises a support plate (30) supporting the reservoir (20), a roller (33) arranged for compressing the reservoir, at least one pinion rack (35) and at least one pinion (34) connected to the roller so that a rotational movement of the pinion (34) on the pinion rack (35) provokes a translational movement of the roller (33) on the reservoir.

4. The automatic medical injection device according to claim 3 in combination with claim 2, wherein the direction of the translational movement of the roller (33) is along the longitudinal axis (X).

5. The automatic medical injection device according to claim 3 or claim 4, wherein the support plate (30) is moveable between:
• a storage position in which the injection needle (25) is maintained inside the body (10), and
• an injection position in which the injection needle (25) is protruding from the body (10) for penetrating the patient's skin,
the body comprising a blocking system configured for selectively maintaining the support plate (30) in the storage position or the injection position.

6. The automatic medical injection device according to claim 5, wherein the support plate (30) is tiltable around a tilting axis (Y_{T}) between the storage position and the injection position.

7. The automatic medical injection device according to any of claims 1 to 6, further comprising at least one holding flap (12) protruding from the upper surface of the body (10), preferably two holding flaps (12), said at least one holding flap (12) being configured for at least partially surrounding a hand of a user during injection.

8. The automatic medical injection device according to any of claims 1 to 7, further comprising a Peltier element (15) adapted for cooling the patient's skin, said Peltier element (15) surrounding the throughhole (14) in the lower surface.

9. The automatic medical injection device of any of claims 1 to 8, further comprising a beeper configured for emitting an acoustic signal at the end of the injection.

10. The automatic medical injection device according to any of claims 1 to 9, wherein the body comprises a microprocessor and a motor, the motor being arranged to drive the compression device so as to expel the medical solution through the needle, said motor being controlled by the microprocessor.

11. The automatic medical injection device according to claim 10, in combination with claim 4, wherein the motor is configured to drive the pinion to roll on the pinion rack.

12. The automatic medical injection device according to claim 10 or claim 11, wherein the activation button is configured for starting and/or stopping the motor and/or controlling a speed of the motor.

13. The automatic medical injection device according to any of claims 8 to 12, further comprising a rechargeable battery adapted for supplying electrical power to at least one of the microprocessor, the motor, the Peltier element (15), or the beeper, the body further comprising an electrical connector (17) arranged through a wall of the body and configured to connect an electric power source for recharging the rechargeable battery.

14. The automatic medical injection device according to claim 13, wherein the electric connector (17) is further configured to connect an external electronic device to the microprocessor.
